# EUROPÄISCHE PATENTSCHRIFT

(11) **EP 0 534 904 B1**
(45) Veröffentlichungstag und Bekanntmachung des Hinweises auf die Patenterteilung: **23.10.1996**
(21) Anmeldenummer: 92810719.2
(22) Anmeldetag: 21.09.1992
(51) Int. Cl.: C07D 401/06, A61K 31/44

(54) **Imidazolylmethyl-Pyridine**
Imidazolylmethyl-pyridines
Imidazolylméthylpyridines

(30) Priorität: 23.09.1991 DE 4131584
(43) Veröffentlichungstag der Anmeldung: 31.03.1993
(73) Patentinhaber: SANDOZ LTD., 4002 Basel (CH); SANDOZ-PATENT-GMBH, 79539 Lörrach (DE); SANDOZ ERFINDUNGEN VERWALTUNGSGESELLSCHAFT M.B.H., 1235 Wien (AT)
(72) Erfinder: Giger, Rudolf Karl Andreas, CH-4132 Muttenz (CH)

(56) Entgegenhaltungen:
- EP-A- 0 003 560
- EP-A- 0 214 740
- EP-A- 0 337 928
- GB-A- 2 028 317

## Beschreibung

Die vorliegende Erfindung betrifft Imidazolylmethyl-pyridine, ihre Herstellung und Anwendung bei der therapeutischen Behandlung.

Die Erfindung betrifft insbesondere Verbindungen der Formel I, 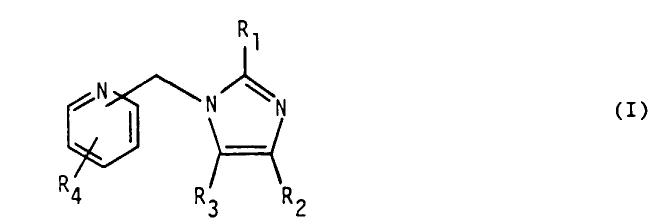 worin
- R₁: Alkyl mit 1 bis 4 Kohlenstoffatomen, Halogen mit einer Atomzahl von 9 bis 35 oder gegebenenfalls durch Alkyl mit 1 bis 4 Kohlenstoffatomen mono- oder disubstituiertes Amino bedeutet,
- R₂ und R₃: unabhängig voneinander für Wasserstoff oder Alkyl mit 1 bis 4 Kohlenstoffatomen stehen und
- R₄: Wasserstoff, Hydroxy, Alkyl oder Alkoxy mit je 1 bis 4 Kohlenstoffatomen oder Halogen mit einer Atomzahl von 9 bis 35 bedeutet,
in Form der freien Basen oder in Säureadditionssalzform, nachstehend als neue Verbindungen bezeichnet.

Insofern oben definierte Alkyl- oder Alkoxygruppen in den neuen Verbindungen vorhanden sind, enthalten diese vorzugsweise 1 oder 2 Kohlenstoffatome und stellen insbesondere Methyl oder Methoxy dar.

Der Imidazolylmethyl-Rest steht bevorzugt in Stellung 2 des Pyridins.

R₁ steht vorzugsweise für Methyl. Bevorzugt bedeuten R₂ und R₃ je Wasserstoff. R₄ steht vorzugsweise für Wasserstoff. Die Verbindung des Beispiels 1 ist bevorzugt.

Die Erfindung umfasst insbesondere eine Gruppe von neuen Verbindungen, worin R₁ Alkyl mit 1 bis 4 Kohlenstoffatomen bedeutet, R₂ und R₃ unabhängig voneinander für Wasserstoff oder Alkyl mit 1 bis 4 Kohlenstoffatomen stehen und R₄ Wasserstoff, Alkyl mit 1 bis 4 Kohlenstoffatomen oder Halogen mit einer Atomzahl von 9 bis 35 bedeutet.

Erfindungsgemäss gelangt man zu den neuen Verbindungen, indem man Verbindungen der Formel II, 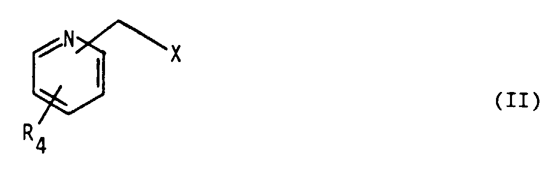 worin R₄ obige Bedeutung besitzt und X für Halogen steht, mit Verbindungen der Formel III, 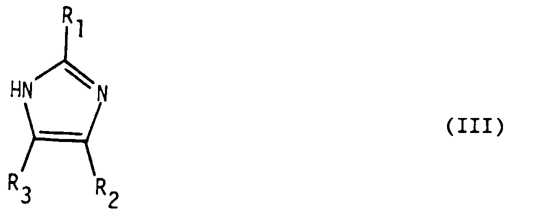 worin R₁, R₂ und R₃ obige Bedeutung besitzen, umsetzt und die erhaltenen Verbindungen der Formel I in Form der freien Basen oder in Säureadditionssalzform gewinnt.

Die Umsetzung der Verbindungen der Formel II mit den Verbindungen der Formel III kann auf an sich bekannter Weise erfolgen, in einem unter den Reaktionsbedingungen inerten Lösungsmittel, z.B. in Dimethylformamid oder einem niederen Alkohol. In der Formel II steht X vorzugsweise für Chlor.

Die Aufarbeitung der erhaltenen Reaktionsgemische und die Reinigung der so gewonnenen Verbindungen der Formel I kann nach an sich bekannten Methoden erfolgen.

Die Verbindungen der Formel I können in freier Form oder in Form ihrer Additionssalze mit Säuren vorliegen. Aus den freien Basen lassen sich in bekannter Weise Säureadditionssalze herstellen und umgekehrt.

Die Ausgangsverbindungen der Formeln II und III sind bekannt oder nach an sich bekannten Verfahren bzw. analog zu an sich bekannten Verfahren herstellbar.

Die Verbindungen der Formel I und ihre physiologisch verträglichen Salze, im Folgenden als erfindungsgemässe Verbindungen bezeichnet, zeichnen sich durch pharmakologische Wirkung aus und sind deshalb verwendbar als Pharmazeutika.

Die erfindungsgemässen Verbindungen bewirken im Schlaf/Wach-Zyklus sowohl bei der freien Ratte wie auch bei der chronisch implantierten Ratte [Methode, siehe J.-M. Vigouret et al., J. Pharmacol 10, 503 (1978)] mit 1 bis 100 mg/kg p.o. eine Vigilanzerhöhung, indem sie eine Verlängerung der Wachphasen hervorrufen.

Ausserdem bewirken die erfindungsgemässen Verbindungen mit 1 bis 100 mg/kg p.o. bei Ratten mit bilateralen Läsionen des Locus coeruleus und des Nucleus basalis Meynert eine signifikante Verbesserung der kognitiven Leistung, die durch die Fähigkeit, einen elektrischen Schock im Schüttelkäfig zu vermeiden, gemessen wird.

Die Methode ist derjenigen von V. Haroutunian et al. [Brain Research 507 (1990) 261 - 266] ähnlich. Männliche OFA Ratten werden mit Pentobarbital anästhesiert und die Läsionen werden in einem stereo-taxischen Apparat mit einem Radiofrequenz-Generator bei 60 ° C während 10 Sekunden erzeugt. Nach 5 Wochen werden die Verhaltensversuche durchgeführt, unter Verwendung eines Schüttelkäfigs gemäss Beschreibung von A.R. Dravid, A.-L. Jaton und E.B. Van Deusen in Experimental Brain Research, Suppl. 13, S. 249 (1986).

Aufgrund dieser Aktivitäten können die erfindungsgemässen Verbindungen zur Behandlung der senilen Demenz, der Alzheimer'schen Krankheit und anderer degenerativen Krankheiten wie Huntington's Chorea, Morbus Parkinson, Steel-Richardson's Syndrom, tardive Dyskinesien, Hyperkinesie, akute Verwirrungszustände, Down's Syndrom, Myasthenia gravis und Friedrich's Ataxie, ferner als Antidepressiva Anwendung finden.

Für diese therapeutische Wirkung beträgt eine empfohlene tägliche Dosis im Bereich von ca. 1 bis 100 mg der Verbindung, die beispielsweise in Teildosen bis 4 mal täglich verabreicht wird.

Die erfindungsgemässen Verbindungen können auf jedem üblichen Weg verabreicht werden, insbesondere enteral, vorzugsweise oral, beispielsweise in Form von Tabletten oder Kapseln oder parenteral, beispielsweise in Form von Injektionslösungen oder Suspensionen.

Die vorliegende Erfindung betrifft ebenfalls therapeutische Zusammensetzungen, die eine erfindungsgemässe Verbindung zusammen mit zumindest einem pharmazeutischen Träger oder Verdünnungsmittel enthalten. Solche Zusammensetzungen können auf an sich bekannte Weise hergestellt werden. Einheitsdosisformen enthalten beipielsweise von ca. 0,25 bis 50 mg einer erfindungsgemässen Verbindung.

Die nachfolgenden Beispiele erläutern die Erfindung. Temperaturangaben erfolgen in Celsiusgraden und sind unkorrigiert.

### BEISPIEL 1: [2-(2-Methylimidazol-1-yl)methyl]pyridin

9,7 g (75 mM) 2-(Chlormethyl)pyridin und 42 g (512 mM) 2-Methylimidazol werden in 40 ml Dimethylformamid suspendiert, dann wird 3 Stunden bei 105 ° gerührt. Das Dimethylformamid wird abdestilliert und der kristalline Rückstand mit Essigester und wenig Hexan verdünnt. Nach Abfiltrieren wird die Mutterlösung eingedampft und das Dimethylformamid abdestilliert, dann mehrmals zwischen Wasser und Methylenchlorid ausgeschüttelt. Man erhält 10,3 g der öligen Titelverbindung.

9,3 g der erhaltenen Base in Ethanol werden mit 12,7 g Fumarsäure versetzt. Das erhaltene Bis(Base)-tris(hydrogenfumarat) kristallisiert aus Ethanol/Essigester und wird 1 x aus Ethanol/Essigester umkristallisiert. Es ist dünnschichtchromatographisch einheitlich und schmilzt bei 109 - 110 °.

Analog zu Beispiel 1 werden folgende [2-(Imidazol-1-yl)methyl]pyridin hergestellt:

| Beispiel | R₁ | R₂ | R₃ | R₄ | Smp. |
|---|---|---|---|---|---|
| 2 | CH₃ | H | H | 6-CH₃ | 129 - 130 ° * |
| 3 | CH₃ | CH₃ | H | H | 250 - 253 ° (Zers.) ** |
| 4 | CH₃ | H | CH₃ | H | 213 - 220 ° (Zers.) ** |

| | | | | | |
|---|---|---|---|---|---|
| * Fumarat | | | | | |
| ** Dihydrochlorid | | | | | |

sowie folgendes [4-(Imidazol-1-yl)methyl]pyridin:

| Beispiel | R₁ | R₂ | R₃ | R₄ | Smp. |
|---|---|---|---|---|---|
| 5 | CH₃ | H | H | H | 155 - 156 ° * |

| | | | | | |
|---|---|---|---|---|---|
| * Fumarat | | | | | |

## Patentansprüche (Patentansprüche für folgende(n) Vertragsstaat(en): AT, BE, CH, LI, DE, DK, FR, GB, IE, IT, LU, NL, PT, SE)

1. Eine Verbindung der Formel I, 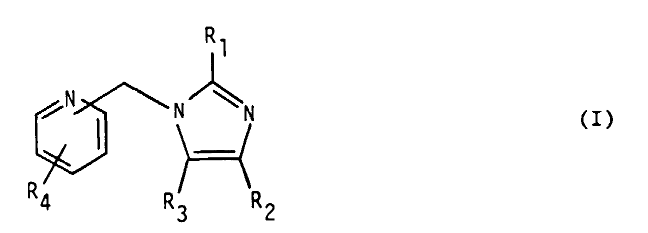 worin
R₁ Alkyl mit 1 bis 4 Kohlenstoffatomen, Halogen mit einer Atomzahl von 9 bis 35 oder gegebenenfalls durch Alkyl mit 1 bis 4 Kohlenstoffatomen mono- oder disubstituiertes Amino bedeutet,
R₂ und R₃ unabhängig voneinander für Wasserstoff oder Alkyl mit 1 bis 4 Kohlenstoffatomen stehen und
R₄ Wasserstoff, Hydroxy, Alkyl oder Alkoxy mit je 1 bis 4 Kohlenstoffatomen oder Halogen mit einer Atomzahl von 9 bis 35 bedeutet,
in Form der freien Base oder in Säureadditionssalzform.

2. Eine Verbindung der Formel I gemäss Anspruch 1, worin R₁ Alkyl mit 1 bis 4 Kohlenstoffatomen bedeutet, R₂ und R₃ unabhängig voneinander für Wasserstoff oder Alkyl mit 1 bis 4 Kohlenstoffatomen stehen und R₄ Wasserstoff, Alkyl mit 1 bis 4 Kohlenstoffatomen oder Halogen mit einer Atomzahl von 9 bis 35 bedeutet, in Form der freien Base oder in Säureadditionssalzform.

3. Eine Verbindung der Formel I gemäss Anspruch 1, worin
- R₁ = CH₃, R₂ = H, R₃ = H, R₄ = 6-CH₃, oder
- R₁ = CH₃, R₂ = CH₃, R₃ = H, R₄ = H, oder
- R₁ = CH₃, R₂ = H, R₃ = CH₃, R₄ = H,
und die Imidazolylmethylgruppe jeweils in Stellung 2 steht, oder
- R₁ = CH₃, R₂ = H, R₃ = H, R₄ = H
und die Imidazolylmethylgruppe in Stellung 4 steht,
in Form der freien Base oder in Säureadditionssalzform.

4. Das [2-(2-Methylimidazol-1-yl)methyl]pyridin in Form der freien Base oder in Säureadditionssalzform.

5. Verfahren zur Herstellung von Verbindungen gemäss Anspruch 1 und ihrer Säureadditionssalze, dadurch gekennzeichnet, dass man Verbindungen der Formel II, 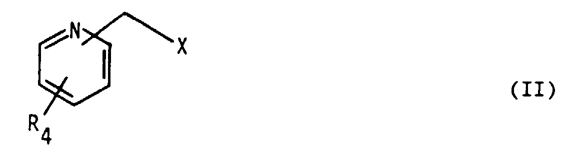 worin R₄ wie im Anspruch 1 definiert ist und X für Halogen steht, mit Verbindungen der Formel III, 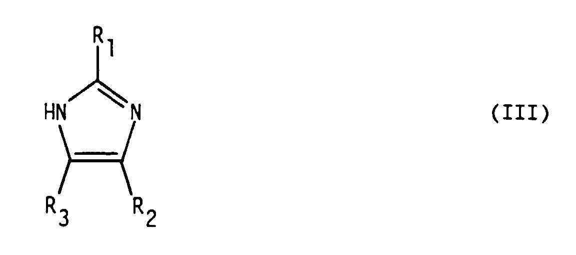 worin R₁, R₂ und R₃ wie im Anspruch 1 definiert sind, umsetzt und die erhaltenen Verbindungen der Formel I in Form der freien Basen oder in Säureadditionssalzform gewinnt.

6. Eine Verbindung nach einem der Ansprüche 1 bis 4, als freie Base oder in physiologisch verträglicher Säureadditionssalzform, zur Anwendung als Pharmazeutikum.

7. Eine Verbindung nach einem der Ansprüche 1 bis 4, als freie Base oder in physiologisch verträglicher Säureadditionssalzform, zur Behandlung der senilen Demenz, der Alzheimer'schen Krankheit, der Huntingtons Chorea, der Morbus Parkinson, des Steel-Richardson's Syndroms, der tardiven Dyskinesien, der Hyperkinesie, der akuten Verwirrungszuständen, des Down's Syndroms, der Myasthenia gravis und der Friedrich's Ataxie.

8. Eine Verbindung nach einem der Ansprüche 1 bis 4, als freie Base oder in physiologisch verträglicher Säureadditionssalzform, zur Anwendung als Antidepressivum.

9. Eine pharmazeutische Zusammensetzung, enthaltend eine Verbindung nach einem der Ansprüche 1 bis 4, als freie Base oder in physiologisch verträglicher Säureadditionssalzform, zusammen mit einem pharmazeutischen Träger oder Verdünnungsmittel.

10. Die Anwendung einer Verbindung nach einem der Ansprüche 1 bis 4, als freie Base oder in physiologisch verträglicher Säureadditionssalzform, für die Herstellung einer pharmazeutischen Zusammensetzung zur Behandlung der senilen Demenz, der Alzheimer'schen Krankheit, der Huntingtons Chorea, der Morbus Parkinson, des Steel-Richardson's Syndroms, der tardiven Dyskinesien, der Hyperkinesie, der akuten Verwirrungszuständen, des Down's Syndroms, der Myasthenia gravis, der Friedrich's Ataxie und zur Anwendung als Antidepressivum.

## Patentansprüche (Patentansprüche für folgende(n) Vertragsstaat(en): ES, GR)

1. Verfahren zur Herstellung von Verbindungen der Formel I, 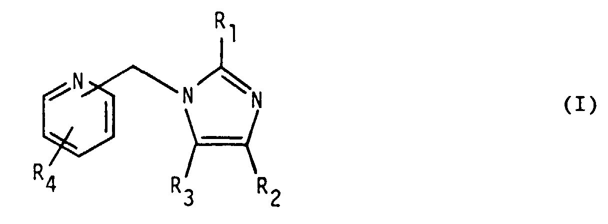 worin
R₁ Alkyl mit 1 bis 4 Kohlenstoffatomen, Halogen mit einer Atomzahl von 9 bis 35 oder gegebenenfalls durch Alkyl mit 1 bis 4 Kohlenstoffatomen mono- oder disubstituiertes Amino bedeutet,
R₂ und R₃ unabhängig voneinander für Wasserstoff oder Alkyl mit 1 bis 4 Kohlenstoffatomen stehen und
R₄ Wasserstoff, Hydroxy, Alkyl oder Alkoxy mit je 1 bis 4 Kohlenstoffatomen oder Halogen mit einer Atomzahl von 9 bis 35 bedeutet,
und ihrer Säureadditionssalze, dadurch gekennzeichnet, dass man Verbindungen der Formel II, 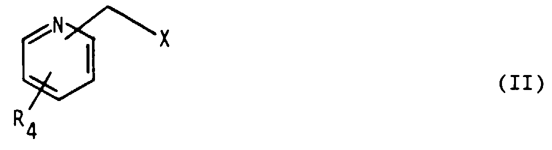 worin R₄ obige Bedeutung besitzt und X für Halogen steht, mit Verbindungen der Formel III, 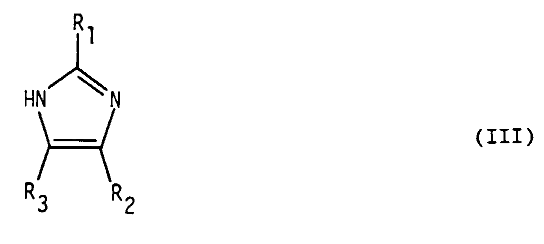 worin R₁, R₂ und R₃ obige Bedeutung besitzen, umsetzt und die erhaltenen Verbindungen der Formel I in Form der freien Basen oder in Säureadditionssalzform gewinnt.

2. Verfahren nach Anspruch 1 zur Herstellung von Verbindungen der Formel I, worin R₁ Alkyl mit 1 bis 4 Kohlenstoffatomen bedeutet, R₂ und R₃ unabhängig voneinander für Wasserstoff oder Alkyl mit 1 bis 4 Kohlenstoffatomen stehen und R₄ Wasserstoff, Alkyl mit 1 bis 4 Kohlenstoffatomen oder Halogen mit einer Atomzahl von 9 bis 35 bedeutet, in Form der freien Basen oder in Säureadditionssalzform.

3. Verfahren nach Anspruch 1 zur Herstellung einer Verbindung der Formel I, worin
- R₁ = CH₃, R₂ = H, R₃ = H, R₄ = 6-CH₃, oder
- R₁ = CH₃, R₂ = CH₃, R₃ = H, R₄ = H, oder
- R₁ = CH₃, R₂ = H, R₃ = CH₃, R₄ = H,
und die Imidazolylmethylgruppe jeweils in Stellung 2 steht, oder
- R₁ = CH₃, R₂ = H, R₃ = H, R₄ = H
und die Imidazolylmethylgruppe in Stellung 4 steht,
in Form der freien Base oder in Säureadditionssalzform.

4. Verfahren nach Anspruch 1 zur Herstellung des [2-(2-Methylimidazol-1-yl)methyl]pyridins in Form der freien Base oder in Säureadditionssalzform.

5. Verfahren zur Herstellung einer pharmazeutischen Zusammensetzung, enthaltend eine Verbindung der Formel I gemäss Anspruch 1 in Form der freien Base oder eines physiologisch verträglichen Säureadditionssalzes, dadurch gekennzeichnet, dass man die Verbindung der Formel I mit einem pharmazeutischen Träger oder Verdünnungsmittel vermischt.

## Claims (Claims for the following Contracting State(s): AT, BE, CH, LI, DE, DK, FR, GB, IE, IT, LU, NL, PT, SE)

1. A compound of formula I, 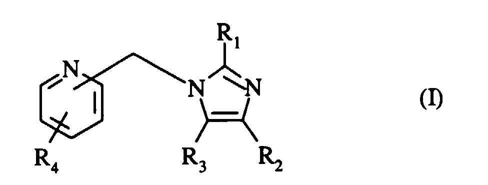 wherein
R₁ is alkyl (1-4 C), halogen with an atomic number of 9 to 35 or amino optionally mono- or disubstituted by alkyl (1-4 C),
R₂ and R₃ independently of one another are hydrogen or alkyl (1-4 C) and
R₄ is hydrogen, hydroxy, alkyl (1-4 C), alkoxy (1-4 C) or halogen with an atomic number of 9 to 35,
in free base or acid addition salt form.

2. A compound of formula I according to claim 1, wherein R₁ is alkyl (1-4 C), R₂ and R₃ independently of one another are hydrogen or alkyl (1-4 C) and R₄ is hydrogen, alkyl (14 C) or halogen with an atomic number of 9 to 35, in free base of acid addition salt form.

3. A compound of formula I according to claim 1, wherein
- R₁ = CH₃, R₂ = H, R₃ = H, R₄ = 6-CH₃, or
- R₁ = CH₃, R₂ = CH₃, R₃ = H, R₄ =H, or
- R₁ = CH₃, R₂ = H, R₃ = CH₃, R₄ =H,
and the imidazolylmethyl group is in position 2, or
- R₁ = CH₃, R₂ = H, R₃ = H, R₄ =H
and the imidazolylmethyl group is in position 4,
in free base or acid addition salt form.

4. [2-(2-methylimidazol-1-yl)methyl] pyridine, in free base or acid addition salt form.

5. A process for the production of compounds according to claim 1 and their addition salts, characterized in that compounds of formula II, 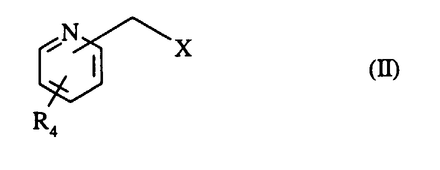 wherein R₄ is as defined in claim 1 and X is halogen, are reacted with compounds of formula III, 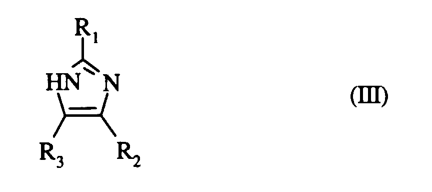 wherein R₁, R₂ and R₃ are as defined in claim 1,
and the resulting compounds of formula I are recovered in free base form or in acid addition salt form.

6. A compound of any one of claims 1 to 4 in free base or physiologically acceptable acid addition salt form, for use as a pharmaceutical.

7. A compound of any one of claims 1 to 4 in free base or physiologically acceptable acid addition salt form, for use in the treatment of senile dementia, Alzheimer's disease, Huntington's chorea, Morbus Parkinson, Steel-Richardson syndrome, tardive dyskinesia, hyperkinesia, acute confusion disorders, Down's syndrome, myasthenia gravis or Friedrich's ataxia.

8. A compound of any one of claims 1 to 4 in free base or physiologically acceptable acid addition salt form, for use as antidepressant.

9. A pharmaceutical composition comprising a compound according to any one of claims 1 to 4 in free base or physiologically acceptable acid addition salt form, in association with a pharmaceutical carrier or diluent.

10. The use of a compound according to any one of claims 1 to 4, in free base or physiologically acceptable acid addition salt form, for the preparation of a pharmaceutical composition for the treatment of senile dementia, Alzheimer's disease, Huntington's chorea, Morbus Parkinson, Steel-Richardson syndrome, tardive dyskinesia, hyperkinesia, acute confusion disorders, Down's syndrome, myasthenia gravis, Friedrich's ataxia and for use as antidepressant.

## Claims (Claims for the following Contracting State(s): ES, GR)

1. A process for the production of compounds of formula I, 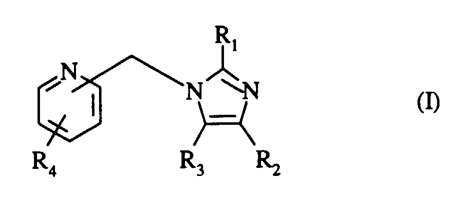 wherein
R₁ is alkyl (1-4 C), halogen with an atomic number of 9 to 35 or amino optionally mono- or disubstituted by alkyl (1-4 C),
R₂ and R₃ independently of one another are hydrogen or alkyl (1-4 C) and
R₄ is hydrogen, hydroxy, alkyl (1-4 C), alkoxy (1-4 C) or halogen with an atomic number of 9 to 35,
and their acid addition salts, characterized in that compounds of formula II, 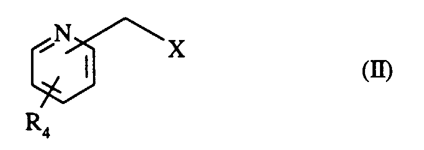 wherein R₄ is defined as above and X is halogen, are reacted with compounds of formula III, 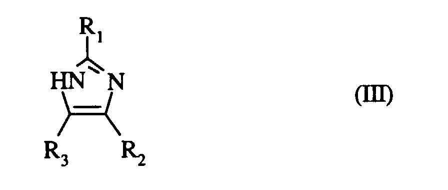 wherein R₁, R₂ and R₃ are as defined above, and the resulting compounds of formula I are recovered in free base form or in acid addition salt form.

2. A process according to claim 1 for the production of compounds of formula I, wherein R₁ is alkyl (1-4 C), R₂ and R₃ independently of one another are hydrogen or alkyl (1-4 C) and R₄ is hydrogen, alkyl (1-4 C) or halogen with an atomic number of 9 to 35, in free base or acid addition salt form.

3. A process according to claim 1 for the production of a compound of formula I, wherein
- R₁ = CH₃, R₂ = H, R₃ = H, R₄ = 6-CH₃, or
- R₁ = CH₃, R₂ = CH₃, R₃ = H, R₄ =H, or
- R₁ = CH₃, R₂ = H, R₃ = CH₃, R₄ =H,
and the imidazolylmethyl group is in position 2, or
- R₁ = CH₃, R₂ = H, R₃ = H, R₄ =H
and the imidazolylmethyl group is in position 4,
in free base or acid addition salt form.

4. A process according to claim 1 for the production of [ 2-(2-methylimidazol-1-yl)methyl]pyridine, in free base or acid addition salt form.

5. A process for the production of a pharmaceutical composition comprising a compound of formula I according to claim 1 in free base or physiologically acceptable acid addition salt form, characterized in that the compound of formula I is mixed with a pharmaceutical carrier or diluent.

## Revendications (Revendications pour l'(les) Etat(s) contractant(s) suivant(s): AT, BE, CH, LI, DE, DK, FR, GB, IE, IT, LU, NL, PT, SE.)

1. Un composé de formule I 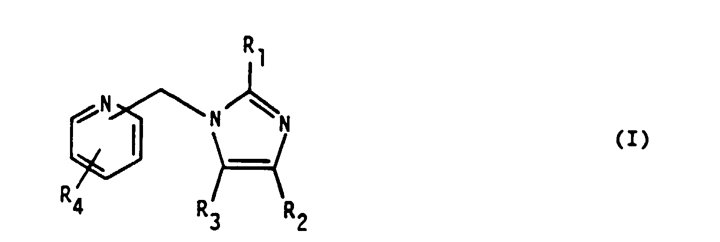 dans laquelle
R₁ signifie un groupe alkyle contenant de 1 à 4 atomes de carbone, un halogène ayant un nombre atomique compris entre 9 et 35 ou un groupe amino éventuellement mono- ou disubstitué par un groupe alkyle contenant de 1 à 4 atomes de carbone,
R₂ et R₃ signifient indépendamment l'un de l'autre, l'hydrogène ou un groupe alkyle contenant de 1 à 4 atomes de carbone, et
R₄ signifie l'hydrogène ou un groupe hydroxy, alkyle ou alcoxy contenant chacun de 1 à 4 atomes de carbone ou un halogène ayant un nombre atomique compris entre 9 et 35,
sous forme de base libre ou sous forme d'un sel d'addition d'acide.

2. Un composé de formule I selon la revendication 1, où R₁ signifie un groupe alkyle contenant de 1 à 4 atomes de carbone, R₂ et R₃ signifient indépendamment l'un de l'autre l'hydrogène ou un groupe alkyle contenant de 1 à 4 atomes de carbone et R₄ signifie l'hydrogène ou un groupe alkyle contenant de 1 à 4 atomes de carbone ou un halogène ayant un nombre atomique compris entre 9 et 35, sous forme de base libre ou sous forme d'un sel d'addition d'acide.

3. Un composé de formule I selon la revendication 1, où
- R₁ = CH₃, R₂ = H, R₃ = H, R₄ = 6-CH₃, ou bien
- R₁ = CH₃, R₂ = CH₃, R₃ = H, R₄ = H, ou bien
- R₁ = CH₃, R₂ = H, R₃ = CH₃, R₄ = H,
et le groupe imidazolylméthyle est situé à chaque fois en position 2, ou bien
- R₁ = CH₃, R₂ = H, R₃ = H, R₄ = H,
et le groupe imidazolylméthyle est situé en position 4,
sous forme de base libre ou sous forme d'un sel d'addition d'acide.

4. La [2-(2-méthylimidazol-1-yl)méthyl]pyridine sous forme de base libre ou sous forme d'un sel d'addition d'acide.

5. Un procédé de préparation des composés selon la revendication 1 et de leurs sels d'addition d'acides, caractérisé en ce qu'on fait réagir les composés de formule II 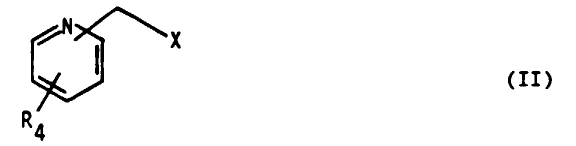 où R₄ est tel que défini à la revendication 1 et X signifie un halogène, avec les composés de formule III 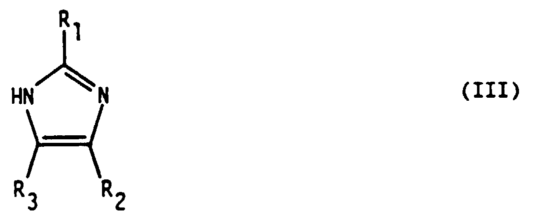 où R₁, R₂ et R₃ sont tels que définis à la revendication 1, et on obtient les composés de formule I sous forme de base libre ou sous forme d'un sel d'addition d'acide.

6. Un composé selon l'une quelconque des revendications 1 à 4, sous forme de base libre ou sous forme d'un sel d'addition d'acide physiologiquement acceptable, pour une utilisation comme médicament.

7. Un composé selon l'une quelconque des revendications 1 à 4, sous forme de base libre ou sous forme d'un sel d'addition d'acide physiologiquement acceptable, pour le traitement de la démence sénile, de la maladie d'Alzheimer, de la chorée de Huntington, de la maladie de Parkinson, du syndrome de Steel-Richardson, de la diskinésie tardive, de l'hyperkinésie, des troubles confusionnels aigus, du syndrome de Down, de la myasthénie grave et de l'ataxie de Friedrich.

8. Un composé selon l'une quelconque des revendications 1 à 4, sous forme de base libre ou sous forme d'un sel d'addition d'acide physiologiquement acceptable, pour l'utilisation comme anti-dépresseur.

9. Une composition pharmaceutique, contenant un composé selon l'une quelconque des revendications 1 à 4, sous forme de base libre ou sous forme d'un sel d'addition d'acide physiologiquement acceptable, ensemble avec un véhicule ou diluant pharmaceutique.

10. L'utilisation d'un composé selon l'une quelconque des revendications 1 à 4, sous forme de base libre ou sous forme d'un sel d'addition d'acide physiologiquement acceptable, pour la préparation d'une composition pharmaceutique pour le traitement de la démence sénile, de la maladie d'Alzheimer, de la chorée de Huntington, de la maladie de Parkinson, du syndrome de Steel-Richardson, de la diskinésie tardive, de l'hyperkinésie, des troubles confusionnels aigus, du syndrome de Down, de la myasthénie grave et de l'ataxie de Friedrich et pour l'utilisation comme anti-dépresseur.

## Revendications (Revendications pour l'(les) Etat(s) contractant(s) suivant(s): ES, GR)

1. Un procédé de préparation des composés de formule I 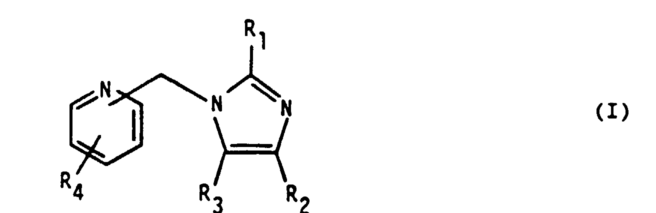 dans laquelle
R₁ signifie un groupe alkyle contenant de 1 à 4 atomes de carbone, un halogène ayant un nombre atomique compris entre 9 et 35 ou un groupe amino éventuellement mono- ou disubstitué par un groupe alkyle contenant de 1 à 4 atomes de carbone,
R₂ et R₃ signifient indépendamment l'un de l'autre, l'hydrogène ou un groupe alkyle contenant de 1 à 4 atomes de carbone, et
R₄ signifie l'hydrogène ou un groupe hydroxy, alkyle ou alcoxy contenant chacun de 1 à 4 atomes de carbone ou un halogène ayant un nombre atomique compris entre 9 et 35,
et leurs sels d'addition d'acides, caractérisé en ce qu'on fait réagir les composés de formule II 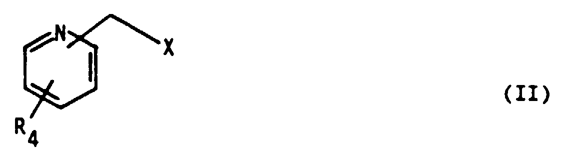 où R₄ est tel que défini plus haut et X signifie un halogène, avec les composés de formule III 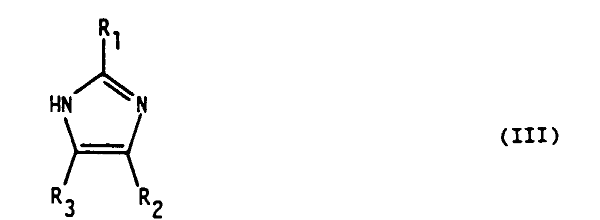 où R₁, R₂ et R₃ sont tels que définis plus haut, et on obtient les composés de formule I sous forme de base libre ou sous forme d'un sel d'addition d'acide.

2. Un procédé selon la revendication 1 pour la préparation des composés de formule I où R₁ signifie un groupe alkyle contenant de 1 à 4 atomes de carbone, R₂ et R₃ signifient indépendamment l'un de l'autre, l'hydrogène ou un groupe alkyle contenant de 1 à 4 atomes de carbone, et R₄ signifie l'hydrogène ou un groupe alkyle contenant de 1 à 4 atomes de carbone ou un halogène ayant un nombre atomique compris entre 9 et 35, sous forme de base libre ou sous forme d'un sel d'addition d'acide.

3. Un procédé selon la revendication 1 pour la préparation d'un composé de formule I où
- R₁ = CH₃, R₂ = H, R₃ = H, R₄ = 6-CH₃, ou bien
- R₁ = CH₃, R₂ = CH₃, R₃ = H, R₄ = H, ou bien
- R₁ = CH₃, R₂ = H, R₃ = CH₃, R₄ = H,
et le groupe imidazolylméthyle est situé à chaque fois en position 2, ou bien
- R₁ = CH₃, R₂ = H, R₃ = H, R₄ = H,
et le groupe imidazolylméthyle est situé en position 4,
sous forme de base libre ou sous forme d'un sel d'addition d'acide.

4. Un procédé selon la revendication 1 pour la préparation de la [2-(2-méthylimidazol-1-yl)méthyl]pyridine sous forme de base libre ou sous forme d'un sel d'addition d'acide.

5. Un procédé de préparation d'une composition pharmaceutique contenant un composé de formule I selon la revendication 1, sous forme de base libre ou sous forme d'un sel d'addition d'acide physiologiquement acceptable, caractérisé en ce qu'on mélange le composé de formule I avec un véhicule ou diluant pharmaceutique.
